(19) Europäisches Patentamt
European Patent Office
Office européen des brevets

(11) **EP 2 070 562 B1**

(12) **FASCICULE DE BREVET EUROPEEN**

(45) Date de publication et mention
de la délivrance du brevet:
**29.12.2010   Bulletin 2010/52**

(51) Int Cl.:
***A61N 1/362*** *(2006.01)*   *A61N 1/365* *(2006.01)*
***A61N 1/368*** *(2006.01)*

(21) Numéro de dépôt: **08291086.0**

(22) Date de dépôt: **19.11.2008**

(54) **Dispositif médical pour la caractérisation de l'état cardiaque d'un patient appareillé avec un implant actif à stimulation biventriculaire**

Medizinische Vorrichtung zur Bestimmung des Herzzustands eines Patienten, dem ein aktives Implantat zur biventrikulären Stimulation eingesetzt wurde

Medical device for characterising the cardiac condition of a patient fitted with an active implant for biventricular stimulation

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **13.12.2007   FR 0708681**

(43) Date de publication de la demande:
**17.06.2009   Bulletin 2009/25**

(73) Titulaire: **Ela Medical**
**92541 Montrouge (FR)**

(72) Inventeurs:
• **Renesto, Fabrizio**
**10013 Borgofranco d'Ivrea (TO) (IT)**

• **Ziglio, Filippo**
**38040 Martignano (TN) (IT)**

(74) Mandataire: **Dupuis-Latour, Dominique et al**
**SEP Bardehle Pagenberg Dost Altenburg Geissler**
**10, boulevard Haussmann**
**75009 Paris (FR)**

(56) Documents cités:
**EP-A- 1 533 001      EP-A- 1 867 360**
**FR-A- 2 887 460      US-A1- 2005 027 320**

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques permettant d'assurer une stimulation conjointe et permanente des ventricules gauche et droit afin de resynchroniser ces derniers, technique dite "CRT" (*Cardiac Resynchronization Therapy*) ou "BVP" (*Bi-Ventricular Pacing*).

En effet, en alternative ou en complément au traitement des troubles du rythme cardiaque, il a été proposé de traiter par stimulation biventriculaire certains troubles de contraction myocardique observés chez des patients en insuffisance cardiaque, ces troubles pouvant être spontanés ou induits par une stimulation traditionnelle. On pourra notamment se référer à l'étude de J. C. Daubert et coll., Stimucoeur, 25, n°3, pp. 170-176 qui fait le point des travaux sur ce sujet. Cette thérapie a permis d'observer des résultats souvent spectaculaires pour des patients en insuffisance cardiaque en classe III non améliorée par les traitements classiques.

Un stimulateur CRT est par exemple divulgué dans le EP 1 108 446 A1 (ELA Medical), qui décrit un dispositif permettant d'appliquer entre les deux stimulations ventriculaires un délai interventriculaire variable, ajusté de manière à resynchroniser la contraction des ventricules avec optimisation fine de l'état hémodynamique du patient.

Les dispositifs CRT utilisés actuellement sont le plus souvent des prothèses dites "multisite" dans lesquelles des électrodes sont placées en une pluralité de sites respectifs distincts comportant au moins un site auriculaire en plus des deux sites ventriculaires droit et gauche, comme dans le cas des prothèses "triple chambre" (double stimulation ventriculaire et détection/stimulation auriculaire droite) ou "quadruple chambre" (double stimulation ventriculaire et double détection/stimulation auriculaire). On trouve également des dispositifs multisite où l'une des sondes ventriculaires est placée dans l'apex ventriculaire droit et l'autre en une position choisie pour optimiser la resynchronisation ventriculaire.

On appellera "sites de stimulation" l'emplacement physique des électrodes intracardiaques par rapport au tissu du myocarde. Ces sites peuvent être choisis au moment de l'implantation par un positionnement approprié des électrodes. D'autre part, lorsque le dispositif comporte plusieurs électrodes placées dans une même cavité, une modification du site de stimulation dans cette cavité est alors possible par une commutation interne du dispositif.

La notion de "séquence de stimulation", quant à elle, se référera d'une part à l'ordre suivant lequel les impulsions de stimulation sont appliquées au coeur (par exemple d'abord oreillette, puis ventricule gauche, puis ventricule droit), et d'autre part aux intervalles de temps séparant l'application de ces impulsions successives. La séquence de stimulation est paramétrée à l'implantation, et peut être si nécessaire modifiée par la suite par des commutations internes appropriées du dispositif et par un ajustement des paramètres de séquencement des impulsions.

On appellera "configuration de stimulation" la combinaison des caractéristiques relatives aux "sites de stimulation" et de celles relatives à la "séquence de stimulation".

Il est nécessaire d'évaluer la pertinence de la configuration de stimulation sélectionnée, car celle-ci conditionne l'efficacité de la thérapie par stimulation biventriculaire. De plus, les effets bénéfiques procurés par cette thérapie peuvent conduire, à terme, à réévaluer la configuration primitive pour éventuellement modifier le choix des sites et/ou le paramétrage de la séquence de stimulation.

Les techniques d'évaluation échographiques, qui doivent être mises en oeuvre en milieu hospitalier et par un personnel qualifié, sont coûteuses et ne peuvent être appliquées aussi souvent que cela serait utile ou nécessaire sans interférer avec la vie quotidienne du patient.

Une solution proposée par le EP 1 108 446 A1 précité consiste à évaluer le degré de synchronisation des contractions des ventricules droit et gauche par une mesure de bioimpédance intracardiaque. Cette donnée est en effet représentative du débit cardiaque et donc de la fraction d'éjection, considérée comme le paramètre hémodynamique de référence.

La présente invention est basée sur une autre approche de l'optimisation de la stimulation biventriculaire, mettant en oeuvre une analyse de l'accélération endocardiaque, plus précisément une analyse des pics d'accélération endocardiaque.

En effet, les études cliniques qui ont été menées indiquent que l'accélération endocardiaque est un paramètre permettant de fournir des informations très complètes sur l'état fonctionnel du myocarde, aussi bien dans le cas d'un fonctionnement normal que d'un fonctionnement déficient : l'accélération endocardiaque, qui est mesurée par un accéléromètre directement en contact avec le muscle cardiaque (généralement, mais non exclusivement, à l'apex ventriculaire droit, parfois dans l'oreillette droite), reflète en effet très précisément et en temps réel les phénomènes concourant au fonctionnement mécanique du coeur.

Plus précisément, le EP 0 515 319 A1 (Sorin Biomedica Cardio SpA) enseigne la manière de recueillir un signal d'accélération endocardiaque au moyen d'une sonde endocavitaire pourvue d'une électrode distale de stimulation implantée au fond du ventricule et intégrant un microaccéléromètre permettant de mesurer l'accélération endocardiaque. Le signal d'accélération endocardiaque ainsi recueilli au cours d'un cycle cardiaque forme deux pics, correspondant aux deux bruits majeurs qu'il est possible de reconnaître dans chaque cycle d'un coeur sain :

- le premier pic d'accélération endocardiaque ("PEA1") correspond à la fermeture des valvules mitrale et tricuspide, au début de la phase de contraction ventriculaire isovolumique (systole). Les variations de ce premier pic sont étroitement liés aux variations de la pression dans le ventricule (l'amplitude du pic PEA1 étant, plus précisément, corrélée au maximum positif de la variation de pression dP/dt dans le ventricule gauche) et peuvent donc constituer un paramètre représentatif de la contractilité du myocarde, elle-même liée au niveau d'activité du système sympathique ;
- le second pic d'accélération endocardiaque ("PEA2"), quant à lui, correspond à la fermeture des valvules aortique et pulmonaire, au moment de la phase de relaxation ventriculaire isovolumique. Ce second pic, produit par la décélération brusque de la masse sanguine en mouvement dans l'aorte, constitue un paramètre représentatif de la pression sanguine périphérique au début de la diastole.

Le EP 0 655 260 A1 (Sorin Biomedica Cardio SpA) décrit une manière de traiter le signal d'accélération endocavitaire délivré par le capteur situé en bout de sonde pour en dériver deux valeurs liées à ces pics d'accélération endocardiaque respectifs, valeurs utiles notamment pour la détection de troubles cardiaques et le déclenchement ou non d'une thérapie de défibrillation.

Le EP 1 867 360 A (publié le 19.12. 2007, postérieurement à la date de priorité du présent brevet) décrit un dispositif délivrant un indice composite représentatif de l'etat clinique du patient, et obtenu à partir de divers paramètres, dont les maxima d'amplitude des pics PEA1 et/ou PEA2 et l'intervalle séparant ces deux pics, dans une configuration de stimulation prédéterminée et non modifiée.

Le EP 1 736 203 A1 (= FR 2 887 460 A1) (ELA Medical) décrit une application spécifique aux implants à stimulation biventriculaire, consistant à utiliser les paramètres liés à l'accélération endocardiaque pour déterminer une configuration de stimulation optimale pour le patient, au moment de l'implantation ou ultérieurement. Diverses mesures sont effectuées pour caractériser le signal PEA, et sont combinées pour donner un indice de performance composite, le choix de la configuration de stimulation finale étant celui qui maximise cet indice de performance.

Le point de départ de la présente invention consiste à utiliser les données d'accélération endocardiaque ainsi recueillies et traitées par l'implant, pour évaluer à un moment donné l'état clinique du patient, c'est-à-dire la réponse de celui-ci à la thérapie de resynchronisation cardiaque.

Un indice d'état clinique permettra en particulier d'assurer un suivi de la fonction ventriculaire sur le long terme, notamment pour évaluer les risques d'apparition d'un épisode d'insuffisance cardiaque.

En d'autres termes, l'invention propose d'utiliser des données déjà calculées par l'implant dans le cadre de l'optimisation de la configuration de stimulation (notamment de la manière décrite dans le EP 1 736 203 A1 (= FR 2 887 460 A1) précité). Ces données seront alors traitées, de la manière que l'on décrira plus bas, pour produire un indice d'état clinique du patient.

L'invention pourra être mise en oeuvre notamment au moyen d'un dispositif externe tel qu'un programmateur du même type que celui utilisé par les praticiens lors des contrôles de routine pour interroger et éventuellement modifier le paramétrage de l'implant.

Mais d'autres formes de mise en oeuvre sont possibles, par exemple de façon interne à l'implant, les données d'état clinique pouvant servir à déclencher une alarme à destination du patient et/ou être transmises à un centre distant chargé d'assurer le suivi des patients à partir de données recueillies par télétransmission.

Dans une forme de mise en oeuvre particulière, l'invention utilise l'une des techniques d'analyse décrites dans le EP 1 736 203 A1 (= FR 2 887 460 A1) précité, consistant à obtenir pour chaque configuration de stimulation une caractéristique PEA/DAV en exécutant un balayage du délai atrioventriculaire (DAV) tout en enregistrant les variations de l'amplitude du pic d'accélération endocardiaque (PEA), généralement le premier pic PEA1. Cette caractéristique peut être obtenue par activation périodique, dans un mode de test déclenché par l'implant. Les résultats obtenus sont, selon l'invention, utilisés au moment de ce test pour déterminer un indice d'état clinique du patient, en plus de la vérification de la configuration de stimulation choisie.

Dans son aspect le plus général, la présente invention concerne un dispositif médical comprenant des moyens de caractérisation d'un état cardiaque d'un patient appareillé avec un implant actif apte à appliquer à ce patient une thérapie de resynchronisation cardiaque par stimulation biventriculaire.

L'implant en question est du type général divulgué par le EP 1 736 203 A1 (= FR 2 887 460 A1) précité, dont les éléments essentiels sont exposés dans le préambule de la revendication 1.

L'invention propose, pour atteindre les buts exposés plus haut, de compléter ce dispositif par les éléments énoncé dans la partie caractérisante de la revendication 1.

Les sous-revendications exposent diverses modalités de mise en oeuvre préférentielles.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La figure 1 est un chronogramme montrant, au cours de trois cycles cardiaques successifs, les variations de l'accélération endocavitaire ainsi que celles de l'électrogramme et de l'électrocardiogramme de surface.

La Figure 2 montre l'allure d'une caractéristique PEA/DAV donnant la variation de l'amplitude du pic d'accélération endocardiaque en fonction du délai atrioventriculaire, et explique la manière de modéliser cette caractéristique sous forme de trois segments successifs.

Les Figures 3a et 3b illustrent deux exemples-types de caractéristique PEA/DAV, présentant respectivement une convergence satisfaisante et une convergence insuffisante.

La Figure 4 illustre les différents niveaux du pic d'accélération endocardiaque obtenus en testant toutes les configurations de stimulation possibles successivement pour un même patient et la valeur moyenne du même paramètre.

Les Figures 5a et 5b sont homologues de la Figure 4, pour illustrer les paramètres relevés à trois mois d'intervalle chez un même patient.

La Figure 6 illustre la manière de déterminer le rapport signal/bruit de la valeur du pic d'accélération endocardiaque.

La Figure 7 est un organigramme montrant la manière dont l'indice d'état clinique du patient est déterminé à partir des différents paramètres mesurés par l'implant.

Les Figures 8 et 9 illustrent, pour deux cas cliniques donnés à titre d'exemple, l'évolution au fil du temps de l'indice calculé par l'invention, comparée à l'évolution clinique déterminée de manière traditionnelle par un praticien.

On va maintenant décrire un exemple de réalisation du dispositif de l'invention.

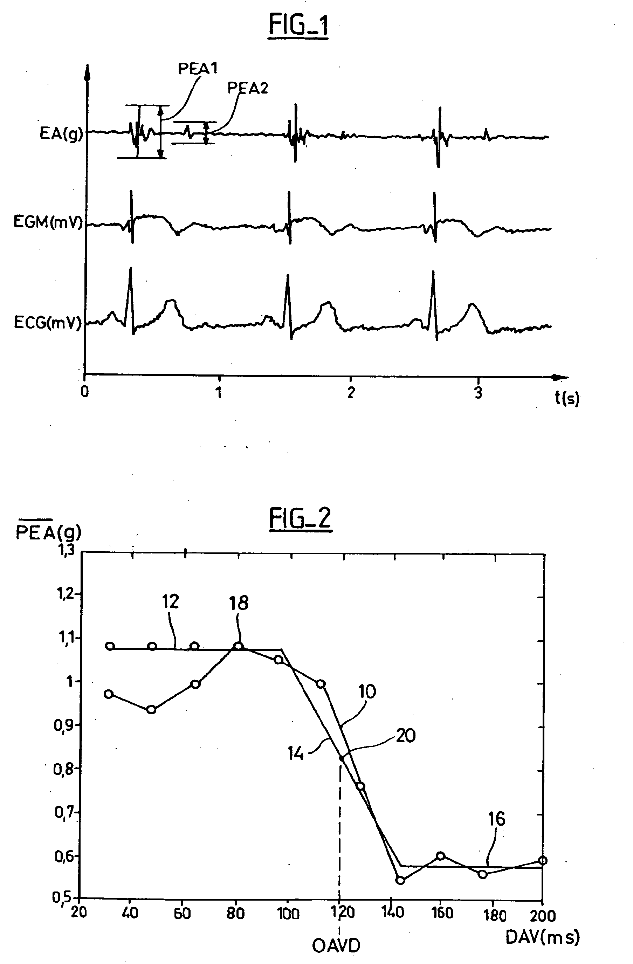

Sur la figure 1, on a représenté (courbe du haut), les variations de l'accélération endocardiaque (EA), mesurée par un capteur tel que celui décrit dans le EP 0 515 319 A1 précité, intégré à une tête de sonde endocavitaire placée au fond du ventricule. On a également illustré sur cette figure les tracés de l'électrogramme (EGM), c'est-à-dire du signal électrique recueilli par l'électrode distale de ce même capteur, et d'un électrocardiogramme de surface (ECG) correspondant, au cours de trois cycles cardiaques consécutifs. Comme on l'a expliqué plus haut, le tracé de l'accélération forme deux complexes ou pics d'accélération endocardiaque (PEA) successifs, dont l'amplitude et la durée peuvent être déterminées par un traitement approprié du signal délivré par le capteur d'accélération, comme cela est décrit dans le EP 0 655 260 A1 précité (par "amplitude du pic" on entendra la valeur maximale crête-à-crête du signal d'accélération séparant les deux extrema, positif et négatif, correspondant aux écarts PEA1 et PEA2 indiqués sur le chronogramme de la figure 1 ; par "durée du pic" on entendra l'intervalle de temps séparant le début et la fin du complexe).

Le EP 1 736 203 A1 précité, auquel on pourra se référer pour plus de détails, décrit la manière d'utiliser certains paramètres liés à l'accélération endocardiaque ainsi recueillie pour déterminer automatiquement une configuration de stimulation optimale pour le patient, aussi bien au moment de l'implantation qu'ultérieurement. Divers paramètres peuvent être utilisés à cette fin, notamment : l'amplitude du PEA1 et/ou du PEA2, la durée du PEA1 et/ou du PEA2, l'intervalle de temps séparant le PEA1 du PEA2 consécutif associé, l'intervalle de temps séparant le PEA2 du PEA1 consécutif du cycle cardiaque suivant.

Dans la description qui va suivre, lorsque l'on fera référence au "PEA" on entendra essentiellement les paramètres liés au premier pic PEA1, qui est généralement le plus significatif. Mais cette caractéristique n'est pas limitative, et l'invention peut également être mise en oeuvre à partir de données relatives au second pic PEA2, ou à une combinaison des données PEA1 et PEA2.

Comme cela est expliqué dans le EP 1 736 203 A1 précité, lors d'une phase spécifique destinée à évaluer et à optimiser la configuration de stimulation, l'implant teste les différentes configurations de stimulation possibles et mesure notamment les amplitudes du PEA obtenues pour chacune de ces configurations. Une autre fonction mise en oeuvre par cet algorithme de test consiste, pour une configuration donnée, à faire varier le délai atrioventriculaire DAV et à recueillir les valeurs correspondantes d'amplitude du PEA. L'algorithme permet ainsi d'ajuster le DAV sur une valeur optimale de délai atrioventriculaire (OAVD) une fois choisie une configuration de stimulation particulière.

Les résultats obtenus lors de ces tests sont, selon l'invention, utilisés pour déterminer un indice d'état clinique du patient. Dans l'exemple que l'on va décrire, cet indice est déterminé à partir des quatre paramètres suivants :

- convergence de l'algorithme de recherche du DAV (possibilité de déterminer un DAV optimal),
- caractère sigmoïde de la caractéristique PEA/DAV,
- moyenne des amplitudes des PEA obtenus avec les différentes configurations de stimulation possibles, et
- (éventuellement) quantification du rapport signal/bruit du PEA.

*Convergence de l'algorithme de recherche du DAV*

En faisant varier le DAV et en relevant les amplitudes du PEA (généralement le PEA1) correspondantes, on obtient une caractéristique PEA/DAV telle que celle référencée 10 sur la Figure 2.

L'amplitude du PEA présente une allure sigmoïde lorsque l'on fait varier le DAV entre deux extrêmes, typiquement entre 30 et 200 ms. On peut interpréter cette courbe en considérant que l'amplitude décroissante du PEA pour des DAV croissants est déterminée par deux facteurs principaux, à savoir :

- la "réserve de contractilité" du myocarde, correspondant au niveau de la ligne de base (valeur limite de PEA pour les DAV longs), et
- le "bruit" produit par les valves cardiaques, principalement la valve mitrale, qui détermine l'élévation du niveau d'amplitude au-dessus de cette ligne de base, pour les DAV les plus courts.

Pour que la seconde composante puisse être significativement présente, il est nécessaire que la première le soit déjà, car c'est la contractilité du myocarde qui est la "force d'entraînement" pour tous les phénomènes mécaniques se produisant pendant le cycle cardiaque.

Cette caractéristique PEA/DAV obtenue lors de la phase de test est analysée par un algorithme destiné à déterminer une valeur optimale du DAV, ci-après désignée OAVD.

Des études cliniques ont montré que la capacité de l'algorithme à calculer une telle valeur OAVD (convergence de l'algorithme) est une indication majeure de la bonne condition ventriculaire du patient.

Essentiellement, il est possible d'obtenir un OAVD (faire converger l'algorithme) si la caractéristique PEA/DAV présente une allure sigmoïde nette, avec une pente marquée entre les valeurs courtes et longues de DAV. Des études cliniques ont en particulier montré une bonne corrélation entre le point milieu de cette courbe et le DAV optimal obtenu par des techniques échocardiographiques traditionnelles.

On va maintenant décrire un exemple d'algorithme permettant d'aboutir à cette valeur optimale OAVD du DAV.

La caractéristique 10 obtenue par recueil des signaux d'amplitude PEA est modélisée sous forme d'une succession de trois segments continus, avec un plateau horizontal 12 correspondant aux DAV inférieurs, un segment central incliné 14 correspondant aux valeurs intermédiaires de DAV et un plateau horizontal 16 correspondant aux valeurs supérieures du DAV.

L'algorithme recherche, parmi les valeurs inférieures de DAV, celle donnant l'amplitude maximale du PEA ; cette valeur est référencée 18 sur l'exemple de la Figure 2. Toutes les valeurs de PEA obtenues pour un DAV inférieur à cette valeur maximale sont alors remplacées par cette dernière valeur, ce qui définit le niveau du plateau supérieur 12.

Les deux autres segments 14 et 16 sont alors déterminés par régression linéaire, d'une manière en elle-même connue, de manière à obtenir l'ajustement le plus étroit entre les trois segments 12, 14 et 16 et la caractéristique réelle obtenue 10.

L'ajustement entre la courbe modélisée (les trois segments) et la caractéristique est évalué au moyen d'un indice de convergence IC, basé sur la régression linéaire qui minimise la somme des restes carrés :

$$IC = 1 - \frac{\chi^2}{\chi^2_{ref}}$$

avec :

$$\chi^2 = \chi^2_{upper} + \chi^2_{lower} + \chi^2_{slope}$$

et :

$$\chi^2_k = \sum_i (Pea_i - k)^2$$

$$\chi^2_{ref} = \sum_N (Pea_N - \overline{Pea})^2$$

Le meilleur ajustement est celui considéré comme procurant la valeur maximale de l'indice de convergence IC. La valeur optimale OAVD du DAV est considérée comme étant celle située au milieu 20 du segment central incliné 14.

L'algorithme procède enfin à la validation de ce délai optimal OAVD, en vérifiant un certain nombre de critères. Il considère qu'un DAV optimal OAVD ainsi obtenu est "valide" si les trois conditions suivantes sont cumulativement vérifiées :

- indice de convergence IC supérieur ou égal à une valeur de seuil donnée, par exemple 0,76 (valeur reflétant un bon ajustement de la courbe modélisée à la caractéristique réelle) ;
- valeur du délai OAVD supérieure à un seuil minimal donné, par exemple OAVD supérieur ou égal à 55 ms ;
- différence de niveau, entre le plateau supérieur 12 et le plateau inférieur 16, supérieure ou égale à 0,15 *g* (ou 10 % de la valeur moyenne de l'amplitude PEA de la caractéristique).

Les valeurs ci-dessus sont bien entendu données uniquement à titre d'exemple ; elles résultent d'études cliniques effectuées pour valider l'invention, mais ne présentent aucun caractère limitatif.

*Caractère sigmoïde de la caractéristique PEA/DAV*

Ce paramètre permet de discriminer, parmi les diverses caractéristiques obtenues, celles présentant un caractère sigmoïde réellement marqué, c'est-à-dire un segment central avec une inclinaison significative, d'avec les courbes plus "plates" dont il n'est pas possible de tirer des indications significatives.

Pour opérer cette distinction, un paramètre dit "facteur sigmoïde" ou "SF" est calculé, à partir des valeurs moyennes d'amplitude PEA obtenues entre les côtés gauche et droit de la caractéristique (on notera que le terme "sigmoïde" doit être entendu dans son acception la plus large, incluant en particulier les situations où la caractéristique est assimilable, comme cela arrive parfois, à un simple droite de pente négative).

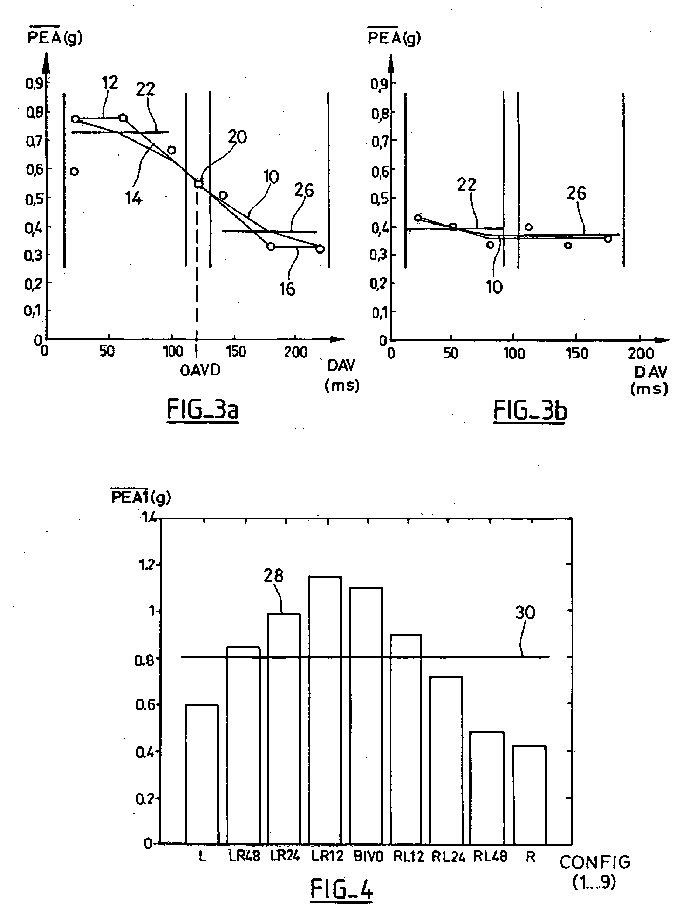

Ces indications sont illustrées graphiquement sur les Figures 3a et 3b, respectivement pour une caractéristique présentant un caractère sigmoïde marqué, et pour une caractéristique de forme beaucoup plus plate.

En effet, dans le cas d'une stimulation biventriculaire, une configuration de stimulation efficace se traduit par une accentuation marquée de la caractéristique PEA /DAV pour les DAV courts, comme dans le cas de la figure 3a. En revanche, en cas d'insuffisance cardiaque, lorsque la réserve de contractilité est marginale la réduction du remplissage ventriculaire pour les DAV les plus courts provoque une chute de contractilité résultant de la loi de Frank-Starling. Pour des DAV courts on obtient alors, comme sur la figure 3b, une augmentation de l'amplitude du PEA produite par le "bruit" des valves cardiaques bien moindre que chez les patients sains, cette augmentation étant quelquefois même à peine perceptible.

La détermination du facteur sigmoïde SF se fait en comparant les niveaux moyens référencés respectivement 22 et 26 sur ces figures. Le calcul est donné par :

$$SF = 1 + \frac{\sum_{i=2}^{3} Pea_i - \sum_{i=5}^{6} Pea_i}{\sum_{i=2}^{3} Pea_i + \sum_{i=5}^{6} Pea_i}$$

Une valeur SF inférieure à l'unité correspond à une courbe inversée, une valeur SF autour de l'unité correspond à une courbe plate, et une valeur SF supérieure à 1 à une courbe présentant l'allure sigmoïde, décroissante recherchée. Plus la valeur SF est élevée, plus le caractère sigmoïde est marqué (ainsi, les deux exemples des Figures 3a et 3b correspondent à des valeurs du facteur sigmoïde respectivement de SF = 1,3831 et SF = 1,0176).

Une évaluation statistique effectuée lors d'études cliniques montre qu'un seuil supérieur ou égal à 1,12 correspond à une valeur significative, avec une sensibilité de 79 % et une spécificité de 81 %.

L'algorithme effectue cette analyse du caractère sigmoïde de la caractéristique PEA/DAV pour toutes les configurations de stimulation possibles et détermine, pour chacune d'entre elles, si le facteur SF dépasse ou non le seuil prédéterminé.

Plus le nombre de caractéristiques répondant à ce critère est grand, plus la possibilité sera élevée d'obtenir une optimisation de la stimulation (sélection de la configuration proprement dite, et ajustement des DAV), avec pour conséquence des chances plus élevées que le patient réponde de façon satisfaisante à la thérapie de resynchronisation.

*Valeurs moyennes des amplitudes PEA*

Un autre paramètre pris en compte par l'algorithme de l'invention est la moyenne des amplitudes des PEA pour toutes les configurations de stimulation possibles.

La Figure 4 illustre les résultats de cette mesure pour neuf configurations possibles, désignées L (stimulation ventriculaire gauche seulement), LR48 (stimulation biventriculaire avec 48 ms de retard gauche-droit), ..., BIV0 (stimulation biventriculaire synchrone), ..., RL48 (stimulation biventriculaire avec 48 ms de retard droite-gauche) et R (stimulation ventriculaire droite uniquement).

Pour chacune des neuf configurations le dispositif mesure la valeur moyenne du premier pic PEA1, représenté en 28 sur la Figure 4. La moyenne de ces neuf valeurs est alors calculée, correspondant au niveau d'amplitude illustré en 30 sur la Figure 4.

Ce paramètre est considéré comme une bonne indication de l'état de contractilité des ventricules, et également comme un signal de défaut éventuel du capteur (en cas de valeur faible ou nulle d'amplitude PEA sur tout ou partie des configurations).

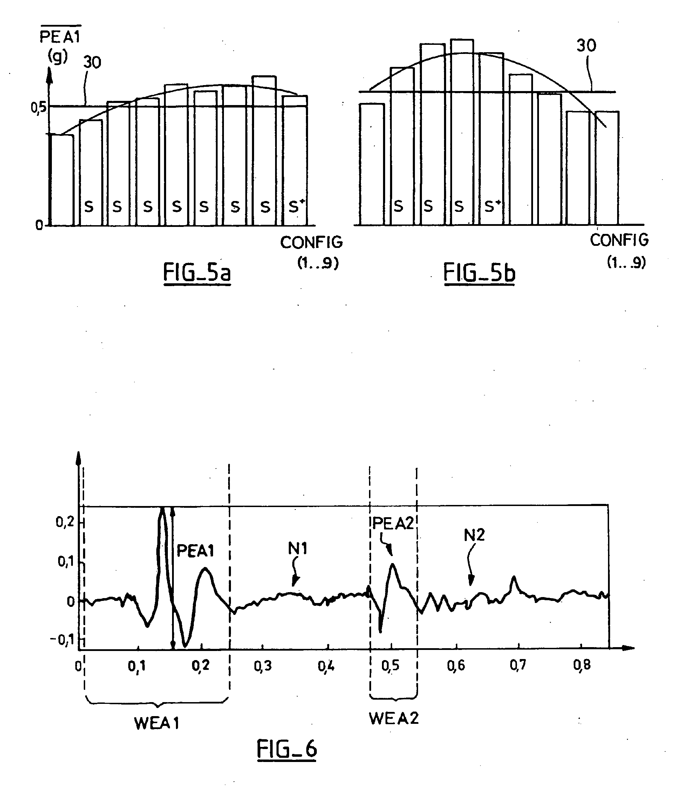

Ainsi, les Figures 5a et 5b, homologues de la Figure 4, montrent la spécificité du paramètre de ce niveau moyen d'amplitude PEA pour deux exemples cliniques différents. Sur ces figures, la lettre "S" indique les configurations pour lesquelles le profil de la caractéristique a été considéré comme présentant un facteur sigmoïde satisfaisant. On voit ainsi que, pour un certain patient (cas de la Figure 5a), bien que l'on obtienne un nombre important de profils sigmoïdes, le niveau moyen 30 du PEA est inférieur à celui d'un autre patient pour lequel le nombre de caractéristiques présentant un facteur sigmoïde satisfaisant est inférieur (Figure 5b).

*Rapport signal/bruit du pic d'accélération endocardiaque*

Un paramètre supplémentaire - et optionnel - est obtenu en quantifiant le rapport signal/bruit du premier pic PEA1, de manière à obtenir une indication de la qualité du signal par rapport aux bruits mécaniques et/ou électriques.

Comme illustré Figure 6, la valeur PEA1 est mesurée à partir de l'amplitude crête à crête de la première composante du signal d'accélération endocardiaque, détecté durant la phase de contraction isovolumique des ventricules, à l'intérieur d'une première fenêtre WEA1. Le niveau de bruit N1 est mesuré lors du même cycle, dans l'intervalle séparant la fin de la fenêtre WEA1 du début de la fenêtre WEA2 correspondant à la composante du second pic PEA2. Le bruit peut également être mesuré en N2 après la fin de la fenêtre WEA2.

Le rapport signal/bruit est donné par :

$$SNR_{PEA1}(n) = \frac{PEA1(n)}{2 \cdot \sigma_{noise}(n)},$$

$\sigma_{noise}(n)$ caractérisant la variabilité du bruit au cours du cycle $n$.

Dans l'exemple de la Figure 6, on obtient ainsi une valeur de rapport signal/bruit SNR de 16,30, pour une valeur de pic PEA1 = 0,397 *g*.

*Détermination de l'état clinique du patient*

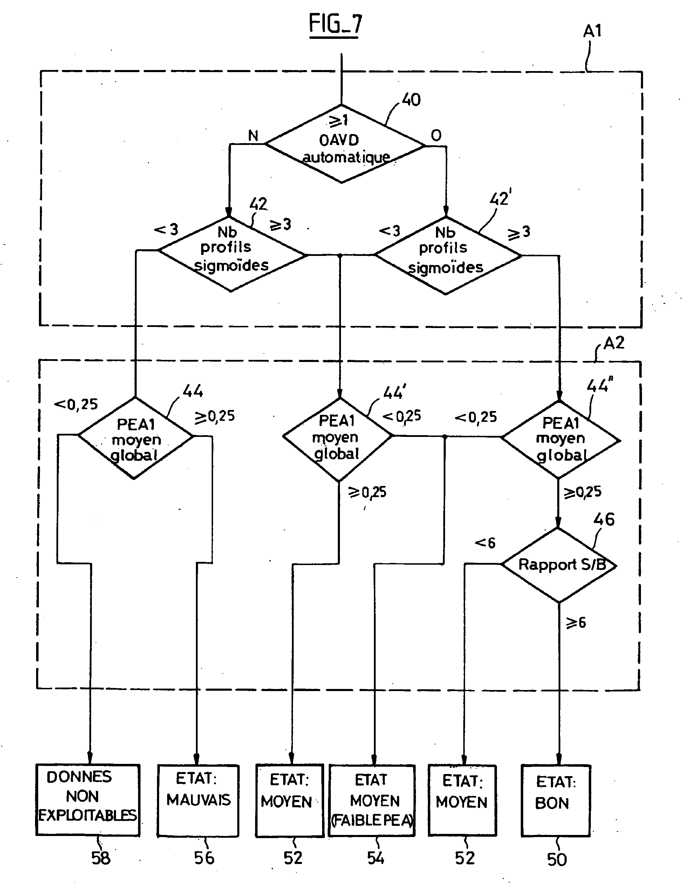

À partir des différents paramètres explicités plus haut, l'algorithme détermine ensuite l'état clinique du patient au moyen d'une table de vérité explicitée par l'organigramme de la Figure 7.

L'analyse s'effectue sur deux niveaux successifs A1 et A2.

Le premier niveau A1 est basé sur les résultats de l'analyse des caractéristiques PEA/DAV : obtention ou non d'un DAV optimal (convergence de l'algorithme), et nombre de profils présentant parmi les différentes configurations un caractère sigmoïde.

L'algorithme vérifie tout d'abord (test 40) qu'au moins un DAV optimal OAVD a été obtenu de façon automatique par l'algorithme, et enchaîne sur une vérification du nombre de profils dont le facteur sigmoïde est supérieur au seuil prédéterminé (SF ≥ 1,2). L'évaluation est "satisfaisante" si, pour les neuf configurations différentes, au moins trois des caractéristiques présentent un profil sigmoïde ; elle est " insuffisante" dans le cas contraire (tests 42, 42').

Le second niveau d'analyse A2 vise à évaluer le niveau général de contractilité, à partir de la moyenne des amplitudes de PEA et (optionnellement) du rapport signal/bruit du PEA.

La valeur moyenne globale du PEA est considérée "satisfaisante" si elle est ≥ 0,25 *g* (tests 44, 44' et 44"), et " insuffisante" dans le cas contraire (mauvaise contractilité ventriculaire, ou défaut du capteur).

En ce qui concerne le rapport signal/bruit, celui-ci sera considéré comme "satisfaisant" si SNR (PEA1) ≥ 6, "insuffisant" dans le cas contraire (reflétant une mauvaise capacité ou fiabilité insuffisante des algorithmes d'optimisation de la configuration de stimulation).

L'état du patient est considéré comme "bon" (résultat 50) pour un patient présentant une optimisation complète (DAV optimal trouvé automatiquement, au moins trois courbes présentant un profil sigmoïde, et avec des niveaux satisfaisants d'amplitude PEA1 et de rapport signal/bruit).

L'état "moyen" (résultat 52) sera attribué à un patient pour lequel l'optimisation est seulement partielle, c'est-à-dire dont l'une seulement des deux conditions ci-dessus du niveau A1 sont satisfaites. Cet état "moyen" est également attribué (résultat 54) à un patient présentant une optimisation complète (les deux critères du niveau d'analyse A1 sont satisfaits) mais avec des signaux dont l'amplitude ou la qualité du PEA est trop faible, traduisant un mauvais niveau de contractilité cardiaque, ou des signaux ne présentant pas une fiabilité suffisante.

L'état "mauvais" (résultat 56) sera attribué à un patient dont aucune des deux conditions du niveau d'analyse N1 n'est vérifiée. Un tel patient ne présente vraisemblablement pas de réponse efficace à la thérapie de resynchronisation, et présente donc un risque élevé d'aggravation de sa pathologie.

Enfin, si le niveau moyen de l'amplitude PEA est très faible, cette situation correspond à des données non exploitables ou, éventuellement, à un problème tel qu'une rupture du capteur (résultat 58).

*Évolution au cours du temps de l'état clinique du patient*

Une fois défini, de la manière exposée plus haut, l'état clinique du patient correspondant à la situation de celui-ci à un moment donné, il est intéressant d'étudier l'évolution de cet état au cours du temps. Ceci en particulier entre deux exécutions successives de l'algorithme d'optimisation de la configuration de stimulation, par exemple à T = 0 lors de l'implantation, et lors de visites de contrôle à T = 3 mois et T = 6 mois.

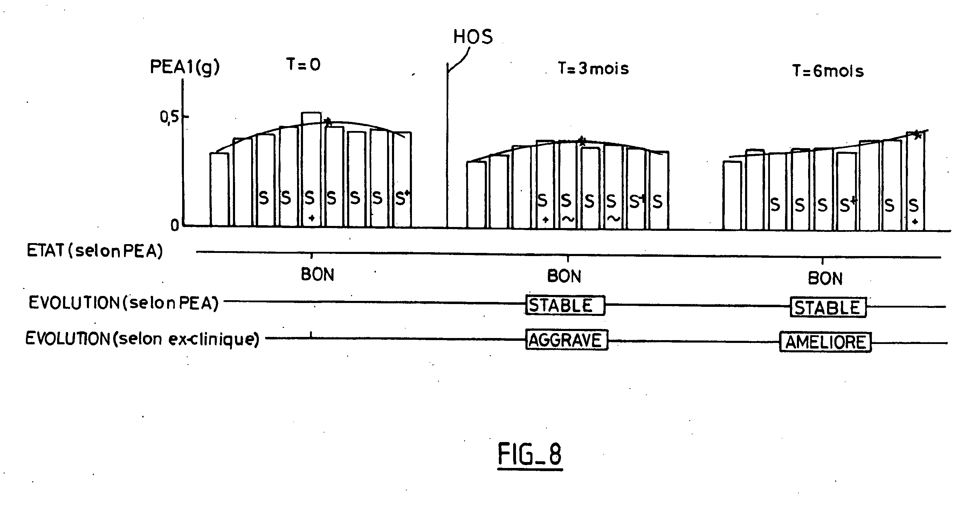

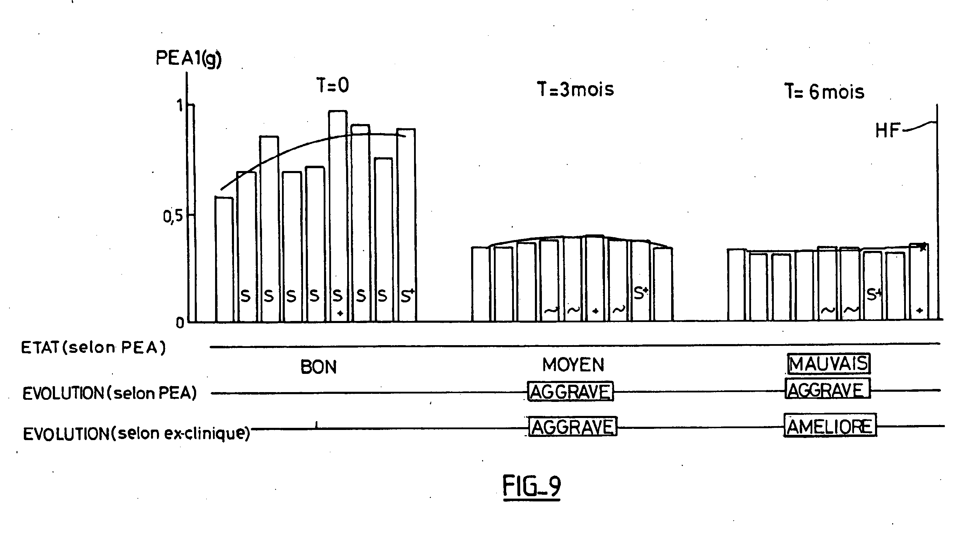

Les Figures 8 et 9 montrent, pour deux cas cliniques différents, l'évolution de ces différents paramètres. Ces figures illustrent, en partie supérieure, les niveaux d'amplitude des pics PEA1 obtenus pour les diverses configurations de stimulation (correspondant aux exemples des Figures 5a et 5b), avec pour chacune l'indication de la présence ou non d'un profil reconnu sigmoïde pour la caractéristique PEA/DAV : la lettre "S" indique que ce critère est vérifié, le symbole "+" indique celle des configurations de stimulation qui a été sélectionnée comme la meilleure par l'algorithme d'optimisation, et le symbole "~" indique des configurations considérées comme à peu près équivalentes à la configuration la meilleure sélectionnée par l'algorithme.

Les lignes suivantes indiquent, à T = 0, T = 3 mois et T = 6 mois, l'état clinique déterminé selon l'invention (état selon PEA) ainsi que son évolution : "stable", "aggravé" ou "amélioré".

Selon l'invention, l'état du patient est considéré comme :

- "amélioré" si, entre deux phases d'optimisation de la configuration de stimulation, l'indice d'état clinique a évolué de : "mauvais" vers "moyen" ou bon", ou de "moyen" vers "bon",
- "stable" si l'indice n'a pas changé et qu'il est resté "moyen" ou "bon",

- "aggravé" (ou "toujours mauvais") si l'indice a évolué de "moyen" à "mauvais", de "bon" à "moyen" ou "mauvais", ou est resté "mauvais".

Il est éventuellement possible d'attribuer une situation "améliorée" si, malgré un même état clinique, l'analyse révèle un nombre accru de caractéristiques présentant une forme sigmoïde et/ou un nombre accru de configurations pour lesquelles il est possible d'obtenir automatiquement un DAV optimal (convergence de l'algorithme).

L'évolution résultant de l'analyse de l'accélération endocardiaque est comparée sur les Figures 8 et 9 à l'évaluation telle qu'elle ressort d'un examen clinique par le praticien, qui n'est pas nécessairement la même. En effet, dans l'exemple la Figure 8, entre T = 0 et T = 3 mois, selon les seules données issues de l'analyse selon l'invention, l'état du patient est resté "stable", alors que le praticien avait formulé un diagnostic d'aggravation (faux positif) suite à une hospitalisation du patient (repérée HOS), en fait non corrélée à sa pathologie cardiaque.

Inversement, dans l'exemple de la Figure 9 l'analyse selon l'invention a permis de détecter une aggravation à T = 6 mois, non diagnostiquée par l'examen clinique traditionnel (faux négatif). L'invention fournit ainsi un prédicteur d'un épisode d'insuffisance cardiaque (repérée HF) susceptible de survenir postérieurement au test réalisé à T = 6 mois.

## Revendications

1. Un dispositif médical comprenant des moyens de caractérisation d'un état cardiaque d'un patient appareillé avec un implant actif apte à appliquer à ce patient une thérapie de resynchronisation cardiaque par stimulation biventriculaire, ledit implant comprenant :

   - des moyens de stimulation biventriculaire ;
   - des moyens de recueil d'un signal d'accélération endocardiaque (EA) ;
   - des moyens, activables périodiquement ou à la demande, d'aide à la recherche d'une configuration de stimulation optimale, comprenant :

     · des moyens de test d'une pluralité de configurations de stimulation différentes par modification des sites de stimulation biventriculaire et/ou de la séquence d'application des impulsions de stimulation aux différents sites et/ou de l'intervalle de temps séparant l'application des impulsions de stimulation aux différents sites, et
     · des moyens de délivrance, pour chaque configuration de stimulation testée, d'une pluralité de paramètres dérivés de l'un et/ou de l'autre des deux pics de l'accélération endocardiaque (PEA1, PEA2) apparaissant respectivement lors de la phase de contraction ventriculaire isovolumique et lors de la phase de relaxation ventriculaire isovolumique ; et

   - des moyens de caractérisation de l'état cardiaque du patient, comprenant :

     · des moyens d'analyse de ladite pluralité de paramètres pour en dériver une pluralité correspondante d'indices spécifiques respectifs, et
     · des moyens de combinaison de ces indices spécifiques en un indice composite d'état clinique du patient, représentatif de la réponse du patient à la thérapie de resynchronisation cardiaque,

   dispositif **caractérisé en ce que** :

   - les moyens de caractérisation comprennent des moyens pour analyser une caractéristique PEA/DAV et en dériver :

     · un indice spécifique indicateur de l'obtention, ou non, d'au moins un délai atrioventriculaire optimal, et
     · un indice spécifique représentatif d'un facteur sigmoïde de ladite caractéristique PEA/DAV ; et

   - les moyens de combinaison comprennent des moyens de comparaison, à un premier niveau (A1), de chacun desdits indices spécifiques à des critères prédéterminés respectifs, et de mise en oeuvre d'une table de vérité donnant de manière univoque une valeur correspondante dudit indice composite d'état clinique.

2. Le dispositif de la revendication 1, dans lequel :

   - les moyens de caractérisation de l'état cardiaque du patient comprennent des moyens aptes à rechercher et valider un délai atrioventriculaire optimal (OAVD) par analyse de ladite caractéristique PEA/DAV (10) pour une pluralité de configurations de stimulation différentes, et
   - ledit indice composite d'état clinique est fonction de l'obtention, ou non, d'au moins un délai atrioventriculaire optimal valide parmi l'ensemble desdites configurations de stimulation différentes.

3. Le dispositif de la revendication 1, dans lequel:

   - les moyens de caractérisation de l'état cardiaque du patient comprennent des moyens de modélisation de ladite caractéristique PEA/DAV (10) en trois segments, avec deux plateaux (12, 16) de part et d'autre d'un segment central avec une pente négative (14), et

- ledit indice composite d'état clinique est fonction de la position relative de ces trois segments.

4. Le dispositif de la revendication 1, dans lequel :

- les moyens de caractérisation de l'état cardiaque du patient comprennent des moyens aptes à déterminer un facteur représentatif du caractère sigmoïde de ladite caractéristique PEA/DAV (10) pour une pluralité de configurations de stimulation différentes, et
- ledit indice composite d'état clinique est fonction dudit facteur sigmoïde.

5. Le dispositif de la revendication 4, dans lequel ledit indice composte d'état clinique est également fonction du nombre de configurations, parmi l'ensemble desdites configurations de stimulation différentes, pour lesquelles ledit facteur sigmoïde respectif dépasse un seuil donné.

6. Le dispositif de la revendication 1, dans lequel :

- les moyens de caractérisation de l'état cardiaque du patient comprennent des moyens aptes à calculer une moyenne (30) des amplitudes (28) des pics d'accélération endocardiaque pour une pluralité de configurations de stimulation différentes, et
- ledit indice composite d'état clinique est fonction de ladite moyenne.

7. Le dispositif de la revendication 1, dans lequel :

- les moyens de caractérisation de l'état cardiaque du patient comprennent des moyens aptes à quantifier le rapport signal/bruit du pic d'accélération endocardiaque, et
- ledit indice composite d'état clinique est fonction du résultat de ladite quantification.

8. Le dispositif de la revendication 1, dans lequel lesdits moyens de comparaison opèrent une comparaison à un second niveau (A2) sur des indices spécifiques comprenant :

- un indice spécifique dérivé d'une moyenne des amplitudes des pics d'accélération endocardiaque ; et
- un indice spécifique dérivé d'une quantification du rapport signal/bruit du pic d'accélération endocardiaque.

9. Le dispositif de la revendication 1, dans lequel les moyens de caractérisation de l'état cardiaque du patient comprennent en outre :

- des moyens d'analyse de l'évolution au cours du temps d'au moins deux valeurs successives dudit indice composite d'état clinique, et
- des moyens pour dériver de cette analyse un indicateur d'évolution de l'état du patient et de risque d'apparition d'un épisode d'insuffisance cardiaque.

**Claims**

1. Medical device comprising means of characterizing a cardiac state of a patient fitted with an active implant capable of applying to this patient a cardiac resynchronization therapy by biventricular stimulation, said implant comprising:

- biventricular stimulation means;
- means of collecting an endocardiac acceleration signal (EA);
- means, which can be activated periodically or on demand, of assisting in the search for an optimum stimulation configuration, comprising:

· means of testing a plurality of different stimulation configurations by modifying the biventricular stimulation sites and/or the sequence of application of the stimulation impulses to the various sites and/or the time interval separating the application of the stimulation pulses to the various sites, and
· means of delivery, for each stimulation configuration tested, of a plurality of parameters derived from one and/or the other of the two endocardiac acceleration spikes (PEA1, PEA2) that appear respectively during the isovolumic ventricular contraction phase and during the isovolumic ventricular relaxation phase; and

- means of characterizing the cardiac state of the patient, comprising:

· means of analysing said plurality of parameters to derive therefrom a corresponding plurality of respective specific indices, and
· means of combining these specific indices into a composite index of the clinical state of the patient, representative of the response of the patient to the cardiac resynchronization therapy,

said device being **characterized in that**:

- the characterization means comprise means for analysing a characteristic PEA/DAV and deriving therefrom:

· a specific index indicating the obtaining of, or failure to obtain, at least one optimal atrioventricular delay, and
· a specific index representative of a signoid factor of said characteristic PEA/DAV; and

- combination means comprising means of comparing, at a first level (A1), each of said specific indices to respective predetermined criteria, and of implementing a truth table unequivocally giving a value corresponding to said composite clinical state index.

2. Device according to Claim 1, in which:

- the means of characterizing the cardiac state of the patient comprise means capable of searching and validating an optimal atrioventricular delay (OAVD) by analysing said characteristic PEA/DAV (10) for a plurality of different stimulation configurations, and
- said composite clinical state index is dependent on obtaining, or failing to obtain, at least one valid optimal atrioventricular delay among all of said different stimulation configurations.

3. Device according to Claim 1, in which:

- the means of characterizing the cardiac state of the patient comprise means of modelling said characteristic PEA/DAV (10) in three segments, with two plateaux (12, 16) either side of a central segment with a negative slope (14), and
- said composite clinical state index is dependent on the relative position of these three segments.

4. Device according to Claim 1, in which:

- the means of characterizing the cardiac state of the patient comprise means capable of determining a factor representative of the sigmoid nature of said characteristic PEA/DAV (10) for a plurality of different stimulation configurations, and
- said composite clinical state index is dependent on said sigmoid factor.

5. Device according to Claim 4, in which said composite clinical state index is also dependent on the number of the configurations, out of all of said different stimulation configurations, for which said respective sigmoid factor exceeds a given threshold.

6. Device according to Claim 1, in which:

- the means of characterizing the cardiac state of the patient comprise means capable of calculating an average (30) of the amplitudes (28) of the endocardiac acceleration spikes for a plurality of different stimulation configurations, and
- said composite clinical state index is dependent on said average.

7. Device according to Claim 1, in which:

- the means of characterizing the cardiac state of the patient comprise means capable of quantizing the signal/noise ratio of the endocardiac acceleration spike, and
- said composite clinical state index is dependent on the result of said quantization.

8. Device according to Claim 1, in which said comparison means apply a comparison at a second level (A2) to specific indices comprising:

- a specific index derived from an average of the amplitudes of the endocardiac acceleration spikes; and
- a specific index derived from a quantization of the signal/noise ratio of the endocardiac acceleration spike.

9. Device according to Claim 1, in which the means of characterizing the cardiac state of the patient also comprise:

- means of analysing the trend over time of at least two successive values of said composite clinical state index, and
- means of deriving, from this analysis, an indicator of the development of the state of the patient and of the risk of the occurrence of a cardiac inadequacy episode.

**Patentansprüche**

1. Medizinische Vorrichtung, die Einrichtungen zur Charakterisierung eines Herzzustands eines Patienten enthält, dem ein aktives Implantat eingesetzt wurde, das an diesem Patienten eine Therapie der kardialen Resynchronisation durch biventrikuläre Stimulation anwenden kann, wobei das Implantat enthält:

- Einrichtungen zur biventrikulären Stimulation;
- Einrichtungen zur Erfassung eines Signals einer endokardialen Beschleunigung (EA);
- periodisch oder nach Bedarf aktivierbare Einrichtungen zur Unterstützung der Suche nach einer optimalen Stimulationskonfiguration, die enthalten:

. Einrichtungen zum Test einer Vielzahl unterschiedlicher Stimulationskonfigurationen durch Änderung der Stellen biventrikulärer Stimulation und/oder der Anwendungsfolge der Stimulationsimpulse an den verschiedenen Stellen und/oder des Zeitintervalls, das die Anwendung der Stimulationsimpulse an den verschiedenen Stellen trennt, und

. Einrichtungen zum Liefern für jede getestete Stimulationskonfiguration einer Vielzahl von Parametern, die von der einen und/oder der anderen der zwei Spitzen der endokardialen Beschleunigung (PEA1, PEA2) abgeleitet werden, die in der Phase der isovolumischen ventrikulären Kontraktion bzw. in der Phase der isovolumischen ventrikulären Relaxation auftreten; und

- Einrichtungen zur Charakterisierung des Herzzustands des Patienten, die enthalten:

. Einrichtungen zur Analyse der Vielzahl von Parametern, um daraus eine entsprechende Vielzahl von jeweiligen spezifischen Indices abzuleiten, und

. Einrichtungen zur Kombination dieser spezifischen Indices in einem Verbundindex des klinischen Zustands des Patienten, der für die Reaktion des Patienten auf die Therapie der kardialen Resynchronisation repräsentativ ist,

wobei die Vorrichtung **dadurch gekennzeichnet ist, dass**:

- die Charakterisierungseinrichtungen Einrichtungen enthalten, um eine PEA/DAV-Charakteristik zu analysieren und daraus abzuleiten:

· einen spezifischen Index, der den Erhalt oder nicht mindestens einer optimalen atrioventrikulären Verzögerung anzeigt, und
· einen spezifischen Index, der für einen sigmoiden Faktor der PEA/DAV-Charakteristik repräsentativ ist; und

- die Kombinationseinrichtungen Einrichtungen zum Vergleich auf einer ersten Ebene (A1) jedes der spezifischen Indices mit jeweiligen vorbestimmten Kriterien und zur Nutzung einer Wahrheitstabelle enthalten, die eindeutig einen entsprechenden Wert des Verbundindex des klinischen Zustands angibt.

2. Vorrichtung nach Anspruch 1, bei der:

- die Einrichtungen zur Charakterisierung des Herzzustands des Patienten Einrichtungen enthalten, die eine optimale atrioventrikuläre Verzögerung (OAVD) durch Analyse der PEA/DAV-Charakteristik (10) für eine Vielzahl unterschiedlicher Stimulationskonfigurationen suchen und validieren können, und
- der Verbundindex des klinischen Zustands vom Erhalt oder nicht mindestens einer gültigen optimalen atrioventrikulären Verzögerung unter der Gesamtheit der verschiedenen Stimulationskonfigurationen abhängt.

3. Vorrichtung nach Anspruch 1, bei der:

- die Einrichtungen zur Charakterisierung des Herzzustands des Patienten Einrichtungen zur Modellbildung der PEA/DAV-Charakteristik (10) in drei Segmenten enthalten mit zwei Plateaus (12, 16) zu beiden Seiten eines zentralen Segments mit einer negativen Neigung (14) und
- der Verbundindex des klinischen Zustands von der relativen Stellung dieser drei Segmente abhängt.

4. Vorrichtung nach Anspruch 1, bei der:

- die Einrichtungen zur Charakterisierung des Herzzustands des Patienten Einrichtungen enthalten, die einen Faktor bestimmen können, der für den sigmoiden Charakter der PEA/DAV-Charakteristik (10) für eine Vielzahl verschiedener Stimulationskonfigurationen repräsentativ ist, und
- der Verbundindex des klinischen Zustands von dem sigmoiden Faktor abhängt.

5. Vorrichtung nach Anspruch 4, bei der der Verbundindex des klinischen Zustands ebenfalls von der Anzahl von Konfigurationen unter der Gesamtheit der verschiedenen Stimulationskonfigurationen abhängt, bei denen der jeweilige sigmoide Faktor einen gegebenen Schwellwert überschreitet.

6. Vorrichtung nach Anspruch 1, bei der:

- die Einrichtungen zur Charakterisierung des Herzzustands des Patienten Einrichtungen enthalten, die einen Mittelwert (30) der Amplituden (28) der Spitzen endokardialer Beschleunigung für eine Vielzahl verschiedener Stimulationskonfigurationen berechnen können, und
- der Verbundindex des klinischen Zustands von dem Mittelwert abhängt.

7. Vorrichtung nach Anspruch 1, bei der:

- die Einrichtungen zur Charakterisierung des Herzzustands des Patienten Einrichtungen enthalten, die das Signal/Rausch-Verhältnis der Spitze endokardialer Beschleunigung quantifizieren können, und
- der Verbundindex des klinischen Zustands vom Ergebnis der Quantifizierung abhängt.

8. Vorrichtung nach Anspruch 1, bei der die Vergleichsmittel einen Vergleich auf einer zweiten Ebene (A2) an spezifischen Indices durchführen, die enthalten:

- einen spezifischen Index, der von einem Mittelwert der Amplituden der Spitzen endokardialer Beschleunigung abgeleitet wird; und
- einen spezifischen Index, der von einer Quantifizierung des Signal/Rausch-Verhältnisses der Spitze endokardialer Beschleunigung abgeleitet wird.

9. Vorrichtung nach Anspruch 1, bei der die Einrichtungen zur Charakterisierung des Herzzustands des Patienten außerdem enthalten:

- Einrichtungen zur Analyse der zeitlichen Entwicklung von mindestens zwei aufeinander folgenden Werten des Verbundindex des klinischen Zustands und
- Einrichtungen, um von dieser Analyse einen Entwicklungsindikator des Zustands des Patienten und der Gefahr eines Auftretens einer Episode von Herzinsuffizienz abzuleiten.

## FIG_1

## FIG_2

FIG_3a

FIG_3b

FIG_4

14

FIG_5a

FIG_5b

FIG_6

## FIG_7

A1

≥1
OAVD
automatique
40

N                                          O

<3          Nb           ≥3                    <3          Nb           ≥3
profils                                        profils
sigmoïdes          42                              sigmoïdes
42'

A2

<0,25                                      <0,25        <0,25
PEA1                      PEA1                       PEA1
moyen                    moyen                      moyen
global         ≥0,25      global                     global
44                        44'                        44"
                         ≥0,25
                                                    ≥0,25

<6
Rapport S/B
46

≥6

| DONNES NON EXPLOITABLES | ETAT: MAUVAIS | ETAT: MOYEN | ETAT MOYEN (FAIBLE PEA) | ETAT: MOYEN | ETAT: BON |
|---|---|---|---|---|---|
| 58 | 56 | 52 | 54 | 52 | 50 |

FIG_8

FIG_9

## RÉFÉRENCES CITÉES DANS LA DESCRIPTION

**Documents brevets cités dans la description**

- EP 1108446 A1
- EP 0515319 A1
- EP 0655260 A1
- EP 1867360 A
- EP 1736203 A1
- FR 2887460 A1
- FR 2887460

**Littérature non-brevet citée dans la description**

- **J. C. Daubert.** *Stimucoeur,* vol. 25 (3), 170-176